# EUROPEAN PATENT SPECIFICATION

(11) **EP 2 730 211 B1**
(45) Date of publication and mention of the grant of the patent: **27.04.2016**
(21) Application number: 13191821.1
(22) Date of filing: 06.11.2013
(51) Int. Cl.: A61B 1/00

(54) **Endoscope device**
Endoskopvorrichtung
Dispositif d'endoscope

(30) Priority: 07.11.2012 JP 2012245589
(43) Date of publication of application: 14.05.2014
(73) Proprietor: Fujifilm Corporation, Minato-ku Tokyo (JP)
(72) Inventor: Kitano, Ryou, Kanagawa (JP)
(74) Representative: Klunker . Schmitt-Nilson . Hirsch

(56) References cited:
- EP-A1- 2 301 419
- EP-A1- 2 371 267
- US-A- 5 966 168
- US-A1- 2007 038 029

## Description

### BACKGROUND

### 1. Technical Field

The present invention relates to an endoscope device.

### 2. Related Art

A conventional endoscope device is equipped with a pan-focus imaging device capable of focusing from a near point to a far point. The imaging device has a wide focusing range of, for example, 6 to 100 mm. In recent years, with a demand to proximately observe an examined object, an endoscope device capable of selectively perform normal observation for a long-distance object point and close observation for a short-distance object point has been proposed, as disclosed in, for example, Patent Literature 1 (JP-B-4819969). According to the endoscope device, the focusing ranges of two modes of a near-focus mode in which a photographing distance is in a range of 2 to 5 mm and a far-focus mode in which the photographing distance is in the range of 5 to 100 mm may be selectively used.

Document US-A-5 966 168 relates to an endoscope apparatus having an objective optical system to focus respectively a near and a far points different in depth and brightness.

### SUMMARY OF INVENTION

In the endoscope device, however, since a distance between a subject and a front end of an insertion portion of an endoscope cannot be determined by the device, a doctor determines the photographing distance based on an experience while viewing an endoscope image. As a result, since the photographing mode cannot but be manually switched, it is complicated to operate the endoscope.

An object of the present invention is to provide an endoscope device capable of automatically switching a photographing mode according to a photographing distance up to a subject as defined in claim 1.

According to the present invention, the photographing mode may be automatically switched according to the photographing distance up to the subject. As a result, observation from a near view to a distant view may be smoothly performed while reducing the manipulation of the endoscope device by an operator.

### BRIEF DESCRIPTION OF DRAWINGS

FIG. 1 is a block configuration diagram of an endoscope device for describing an embodiment of the present invention.
FIG. 2 is an external appearance view as one example of the endoscope device illustrated in FIG. 1.
FIG. 3 is an enlarged view of an endoscope front end.
FIG. 4 is a graph illustrating an MTF distribution of a predetermined frequency by a square wave chart to an object surface distance up to an observation target.
FIG. 5 is a graph schematically illustrating a relationship between a photographing distance and an opening level of a diaphragm.
FIGS. 6A and 6B illustrate photographed images obtained by photographing return light when illumination light is emitted toward a white flat panel from the endoscope front end having a pair of illumination windows illustrated in FIG. 3 in which FIG. 6A is an explanatory view illustrating a photographed image when the photographing distance is 3 mm and FIG. 6B is an explanatory view illustrating a photographed image when the photographing distance is 7 mm.
FIG. 7 is an explanatory view illustrating a method of estimating a photographing distance from a luminance distribution of a photographed image.
FIG. 8 is a graph illustrating a relationship between an examination line location and luminance by taking a curve D1 of the luminance distribution illustrated in FIG. 7 as one example.
FIG. 9 is a flowchart illustrating a mode switching sequence of a focus distance.
FIG. 10 is a graph illustrating a relationship between a lighting time of a light source and illuminance.
FIG. 11 is a graph illustrating a luminance distribution for the examination line when the number of the illumination windows is one.
FIG. 12A is a configuration view of a light source device having a laser light source and FIG. 12B is a configuration diagram of a light source device having an LED.

### DESCRIPTION OF EMBODIMENTS

Hereinafter, an embodiment of the present invention will be described in detail with reference to the drawings.

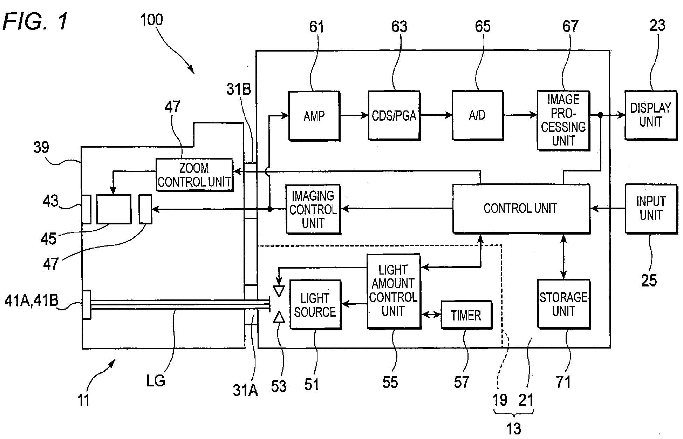

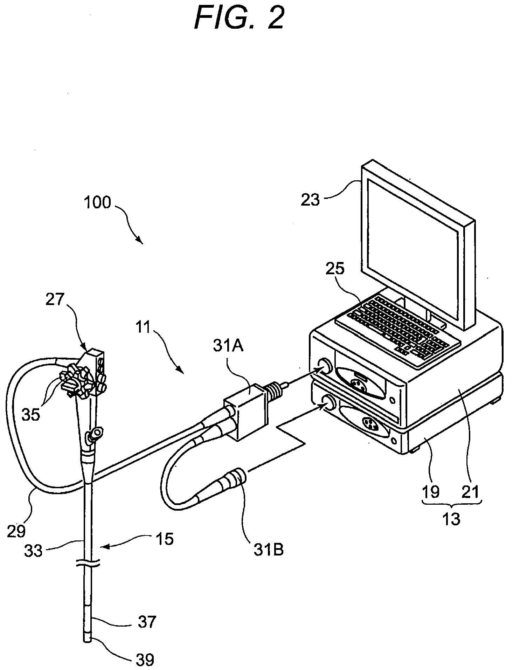

FIG. 1 is a drawing for describing an embodiment of the present invention. FIG. 1 is a block configuration diagram of an endoscope device. FIG. 2 is an external appearance view as one example of the endoscope device illustrated in FIG. 1.

As illustrated in FIGS. 1 and 2, an endoscope device 100 includes an endoscope 11 and a control device 13 to which the endoscope 11 is connected. The endoscope 11 is an electronic endoscope including an illumination optical system that emits illumination light from a front end of an endoscope insertion portion 15 (see FIG. 2) inserted into an examined object and an imaging optical system that includes an imaging element 17 (see FIG. 1) imaging an observed region.

The control device 13 includes a light source device 19 that generates illumination light and a processor 21 that image-processes an image signal from the imaging element 17. A display unit 23 that displays image information or the like, and an input unit 25 that receives an input operation are connected to the control device 13.

As illustrated in FIG. 2, the endoscope 11 includes an endoscope insertion portion 15 inserted into a body cavity, an operating unit 27 that performs a bending operation of a front end of the endoscope insertion portion 15 or an operation for observation, and connector portions 31A and 31B installed on a front end of a universal cord that extends from the operating unit 27 to detachably connect the endoscope 11 to the control device 13. Although not illustrated, various channels such as a forceps channel for inserting a tissue collection treatment tool or the like or air delivery and water delivery channels are provided in the operating unit 27 and the endoscope insertion portion 15.

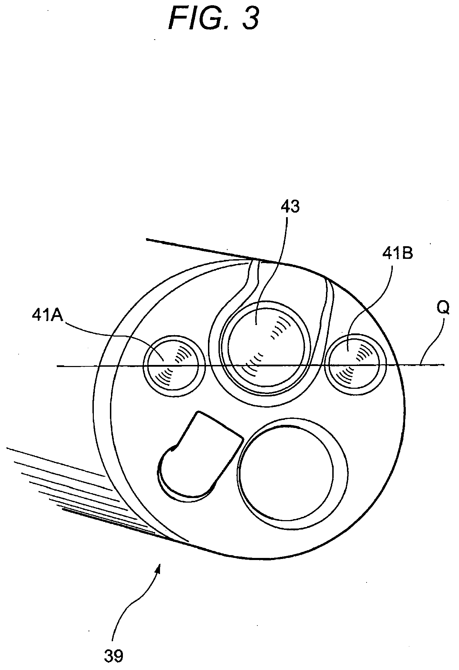

The endoscope insertion portion 15 includes a flexible portion 33 having flexibility, a bending portion 37 which is bendable by a rotating operation of an angle knob 35, and a front end (hereinafter, referred to as an "endoscope front end") 39. As illustrated in an enlarged view of the endoscope front end of FIG. 3, illumination windows 41A and 41B configured to emit the illumination light and an observation window 43 configured to observe a subject are disposed at the endoscope front end 39.

At a position of an optical axis of the observation window 43 in the endoscope front end 39 illustrated in FIG. 1, an objective lens unit 45 that has a zoom tool with a variable focus distance and forms an observation image of the subject is disposed. The imaging element 17 such as a charge coupled device (CCD) image sensor or a complementary metal-oxide semiconductor image sensor (CMOS) as an imaging unit that outputs a photographed image by photographing the observation image obtained from the objective lens unit 45 is disposed at an image-forming side of the observation image in the objective lens unit 45. A zoom control unit 47 sets the zoom tool of the objective lens unit 45 at a desired focus distance (focusing range).

The light source device 19 includes a light source 51 such as a xenon lamp or a halogen lamp, a diaphragm 53 that controls light from the lights source 51, and a light amount control unit 55 the controls the amount of illumination light supplied to the illumination windows 41A and 41B by driving the light source 51 and the diaphragm 53. Further, the light source device 19 has a timer that measures a lighting time of the light source 51 to take out total lighting time information of the light source 51 at any time.

The processor 21 image-processes an imaging signal transmitted from the endoscope 11 based on an instruction from the operating unit 27 or the input unit 25 of the endoscope 11 to generate and supply a displaying image to the display unit 23.

The processor 21 includes an amplifier (hereinafter, referred to as an "AMP") 61 connected to the imaging element 17, a correlation double sampling/programmable gain amplifier (hereinafter, referred to as a "CDS/PGA circuit unit") 63, an A/D converter 65, and an image processing unit 67. Further, the processor 21 includes an imaging control unit 69 that controls the imaging element 17, a storage unit 71 that stores various information tables, and a control unit 73 that generally controls all the units.

The AMP 61 amplifies the imaging signal output from the imaging element 17 with a predetermined gain and outputs the amplified signal to the CDS/PGA circuit unit 63. The CDS/PGA circuit unit 63 outputs the imaging signal input from the AMP 61 as image data of R, G, and B corresponding to charge accumulation amounts of respective light receiving cells of the imaging element 17, and amplifies the image data and outputs the amplified image data to the A/D converter 65. The A/D converter 65 converts analog image data input from the CDS/PGA circuit unit 63 into digital image data and outputs the digital image data to the image processing unit 67. The image processing unit 67 performs various image processings of the image data digitalized by the A/D converter 65 and outputs an observation image in a body cavity to the display unit 23. Further, the image processing unit 67 outputs luminance information of the image data of the photographed image to the control unit 73.

The endoscope device 100 configured as described above is configured to selectively perform normal observation for a long-distance object point and close observation for a short-distance object point, and the control unit 73 illustrated in FIG. 1 controls the zoom control unit 47 according to a photographing distance which is a distance from the observation window 43 up to the subject so as to change a focus distance of the objective lens unit 45. As a result, focusing ranges of two modes of a near-point focusing range mode in which the photographing distance is in the range of 2 to 5 mm and a far-point focusing range mode in which the photographing distance is in the range of 5 to 100 mm are automatically switched.

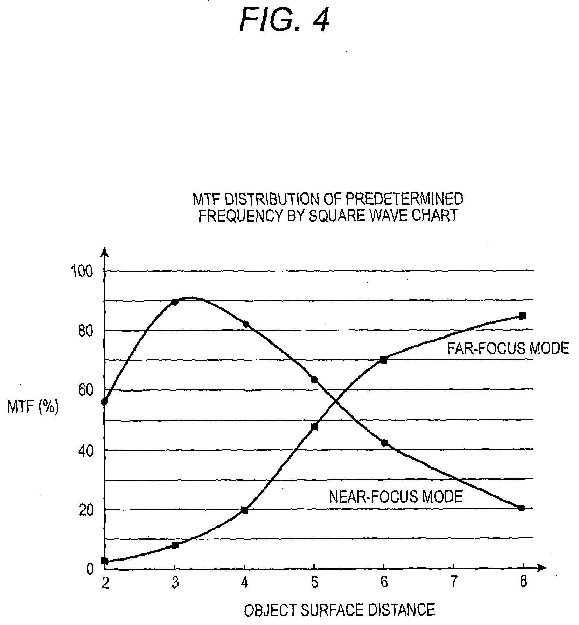

FIG. 4 illustrates an MTF distribution of a predetermined frequency by a square wave chart for an object surface distance up to an observation target. In the far-focus mode, contrast increases when the object surface distance is in a range of 5 mm or more and in the near-focus mode, the contrast increases when the object surface distance is in the range of 2 mm to 5 mm. As a result, when the photographing distance up to the observation target is short as 5 mm or less, the near-focus mode is set and when the photographing distance up to the observation target is long as 5 mm or more, the far-focus mode is set to continuously obtain a high-resolution focusing image.

The control unit 73 obtains the photographing distance by estimation according to light amount information of the illumination light and luminance distribution information of the photographed image output from the imaging element 17. Further, the control unit 73 performs switching to any one of a far-focus mode in which the focus distance of the objective lens unit 45 is set at an focusing location of a subject which is farthest from the observation window 43 and a near-focus mode in which the focus distance of the objective lens unit 45 is set at an focusing location of a subject which is closest to the observation window 43, according to the estimated photographing distance. That is, the control unit sets the focus distance of the objective lens unit 45 to be in a far-point focusing range including the focusing location of the subject which is farthest in the far-focus mode and a near-point focusing range including the focusing location of the subject which is closest in the near-focus mode.

For example, when the far-focus mode is set, the subject may be observed in the photographing distance of 5 mm or more. In this case, when an operator moves the endoscope front end closer to the subject to observe the subject in the photographing distance which is less than 5 mm, the focusing cannot be achieved in the far-focus mode, and as a result, observation is disabled. In this case, the control unit 73 serves as a focus distance switching unit to automatically switch the far-focus mode to the near-focus mode. In the near-focus mode, since the subject may be observed in the photographing distance of 2 to 5 mm, the observation window and the subject may be focused even if the observation window and the subject are close to each other in the range of 2 mm.

On the contrary, when the near-focus mode is set and the operator moves the endoscope front end away from the subject and observes the subject in the photographing distance which exceeds 5 mm, the observation window and the subject cannot be focused, and as a result, the observation is disabled. In this case, the control unit 73 automatically switches the near-focus mode to the far-focus mode to obtain the focusing image.

Hereinafter, a method of estimating the photographing distance will be described.

### (1) Estimation of photographing distance by light amount information of illumination light

The light amount control unit 55 obtains a return light amount of the illumination light from the luminance information of the photographed image output from the image processing unit 67 to control the diaphragm 53 according to the return light amount. The light amount control unit 55 controls the diaphragm 53 to be closed when the amount of return light is large and opened when the amount of the return light is small.

Therefore, the control unit 73 reads a control state of the diaphragm of the light amount control unit 55 and determines that the subject is at a close location when the amount of the return light is large and an opening level of the diaphragm is small and determines that the subject is at a distant location when the amount of the return light is small and the opening level of the diaphragm is large. A threshold value (second threshold value) of the opening level of the diaphragm which is a reference for determining the photographing distance up to the subject in this caseis stored in the storage unit 71 in advance. Further, the opening level of the diaphragm has the same meaning as a light amount of the used illumination light when an emitting intensity of the light source 51 is constant as configured above.

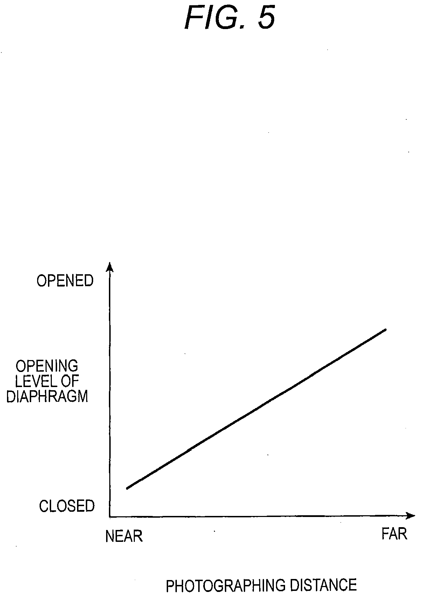

FIG. 5 is a graph schematically illustrating a relationship between a photographing distance and an opening level of a diaphragm. The photographing distance may be estimated from the diaphragm opening level of the diaphragm 53, that is, the light amount of the used illumination light corresponding to the opening level of the diaphragm controlled by the light amount control unit 55. Accordingly, in this configuration, the light amount of the used illumination light in which the photographing distance is 5 mm may be set to the threshold value in which the switching of the near-focus mode and the far-focus mode is performed.

### (2) Estimation of photographing distance by luminance distribution information of photographed image output from imaging element 17

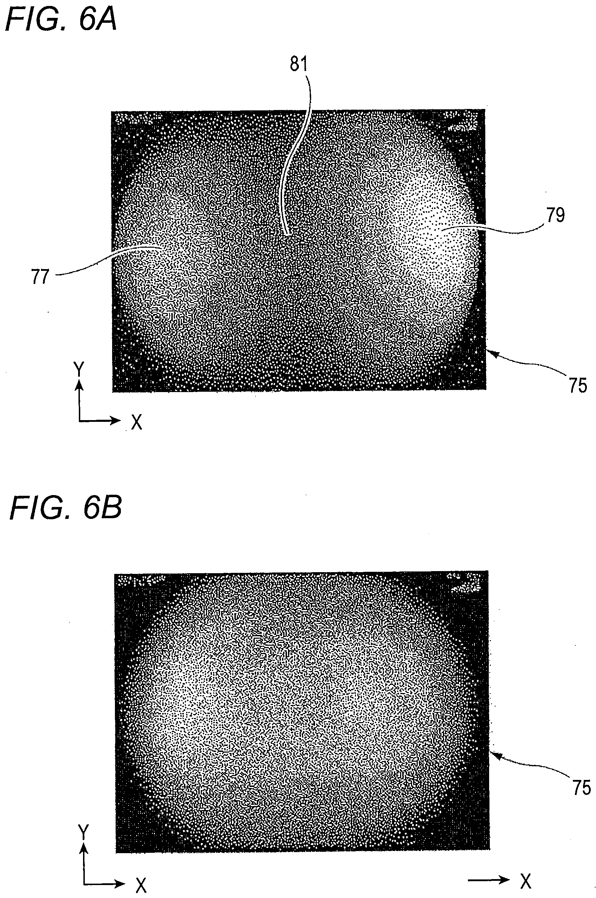

FIGS. 6A and 6B illustrate photographed images obtained by photographing return light when illumination light is emitted toward a white flat panel from the endoscope front end 39 having a pair of illumination windows 41A and 41B illustrated in FIG. 3, and FIG. 6A illustrates a photographed image when the photographing distance is 3 mm and FIG. 6B illustrates a photographed image when the photographing distance is 7 mm.

As illustrated in FIG. 6A, when the photographing distance is short, an x direction corresponding to a straight line Q (equivalent to an x direction) indicating arrangement directions of the illumination windows 41A and 41B and the observation window 43 at the endoscope front end 39 illustrated in FIG. 3 is illustrated, and a peak and a valley of luminance are generated in a luminance distribution. That is, a luminance peak 77 corresponding to the illumination window 41A and a luminance peak 79 corresponding to the illumination window 41B are illustrated in the photographed image 75, and a region of a valley 81 in which luminance decreases is illustrated between the luminance peaks 77 and 79.

Meanwhile, as illustrated in FIG. 6B, when the photographing distance is long, the peak or the valley of the luminance in which the light amount decreases is not clearly illustrated and a substantially equivalent light amount distribution is generated.

As described above, a distinct characteristic occurs in the luminance distribution of the photographed image depending on the change of the photographing distance. The photographing distance when the photographed image is photographed may be estimated based on a characteristic amount of the luminance distribution.

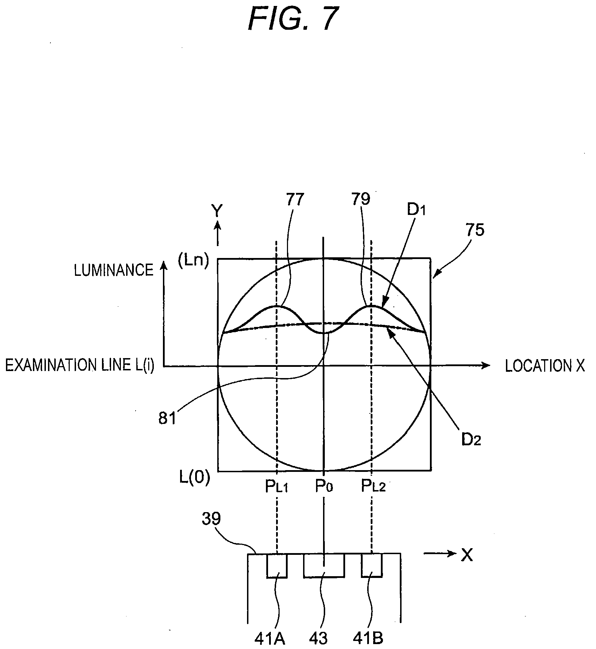

FIG. 7 is an explanatory view illustrating a method for estimating the photographing distance from the luminance distribution of the photographed image. As illustrated in FIG. 7, characteristic amounts appear at a location P_{L1} in the x direction corresponding to the illumination window 41A of the endoscope front end 39, a location P_{L2} in the x direction corresponding to the illumination window 41B, and a location P₀ (a center location of the photographed image) corresponding to the observation window 43, in the photographed image 75.

That is, when the photographing distance is short, the luminance peak 77, the luminance peak 79, and the luminance valley 81 appear at the location P_{L1}, the location P_{L2}, and the location P₀, respectively, as indicated by a solid line D1 in the figure. When the photographing distance is long, a clear peak or valley does not appear and the luminance distribution becomes flat, as indicated by a chain double-dashed line D2 in the figure.

Further, the curves of the illustrated luminance distribution (the solid line D1 and the chain double-dashed line D2) represent a luminance value on an examination line L(i) by using a line along the x direction through the center location O of the photographed image 75 as the examination line L(i). Herein, the L(i) represents the iₜₕ line from the bottom in the figure among n lines following the x direction of the photographed image 75 and arranged in the y direction.

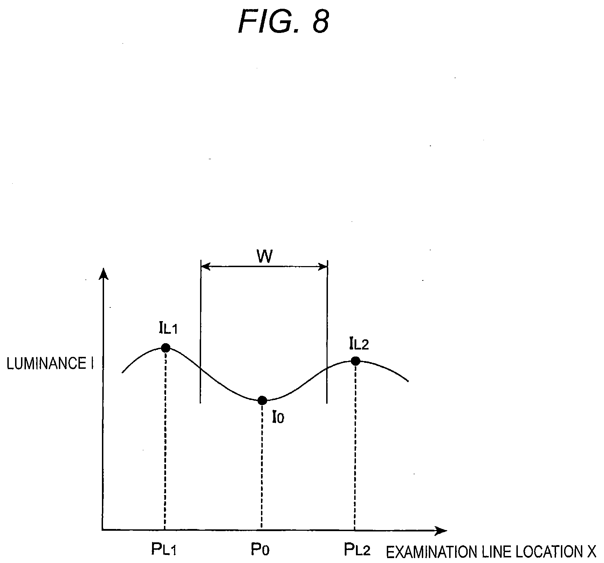

FIG. 8 is a graph illustrating a relationship between an examination line location and luminance by taking the curve D1 of the luminance distribution illustrated in FIG. 7 as an example. The curve D1 has maximum points I_{L1} and I_{L2} at the locations P_{L1} and P_{L2}, respectively. Further, the curve D1 has a minimum point I₀ within a predetermined range W around a pixel including the location P₀ in the examination line.

Therefore, the control unit 73 obtains a luminance ratio (I_{L1}/I₀) of the maximum point I_{L1} to the minimum point I₀ of the curve D1 and a luminance ratio (I_{L2}/I₀) of the maximum point I_{L1} to the minimum point I₀ to compare each luminance ratio with a predetermined threshold value (first threshold value). When at least one luminance ratio of the maximum points I_{L1} and I_{L2} is equal to or more than the threshold value, it is determined that the subject is at the near-focus location and when the luminance ratio is less than the threshold value, it is determined that the subject is at the far-focus location. The threshold value (first threshold value) of the luminance ratio which is a reference for determining whether the subject is at the near-focus location or at the far-focus location is stored in the storage unit 71 in advance. Further, the control unit 73 sets the predetermined range W as a detection range to a range including the center location P₀ from between the location P₀ and the location P_{L1} to between the location P₀ and the location P_{L2} when detecting the minimum point on the examination line to exclude both x-direction ends of the photographed image.

In such a method, it is determined whether the subject is at the near-focus or at the far-focus based on luminance ratios at three spots on the curve D1. However, when it is determined by the minimum point I₀ and any one maximum point, determination processing may be simplified.

The curve D1 (similarly for D2) for acquiring the luminance ratio is the luminance distribution of the examination line L(i), but is not limited to a luminance distribution of one line and may use a luminance distribution obtained by an average value of a plurality of lines or a luminance distribution in which a proximity of the center of the photographed image is weighted and synthesized. In this case, since an influence by noise may be decreased, determination precision may be improved.

By comparison with the respective threshold values illustrated in (1) and (2) above, the control unit 73 estimates the photographing distance up to the subject to determine whether the objective lens unit 45 is set in the near-focus mode or the far-focus mode. The control unit 73 outputs a signal for setting the objective lens unit 45 in the decided mode to the zoom control unit 47, and as a result, the lens of the objective lens unit 45 moves in an optical axis direction to switch the focus distance. The method of acquiring the characteristic amount is an example and is not limited thereto.

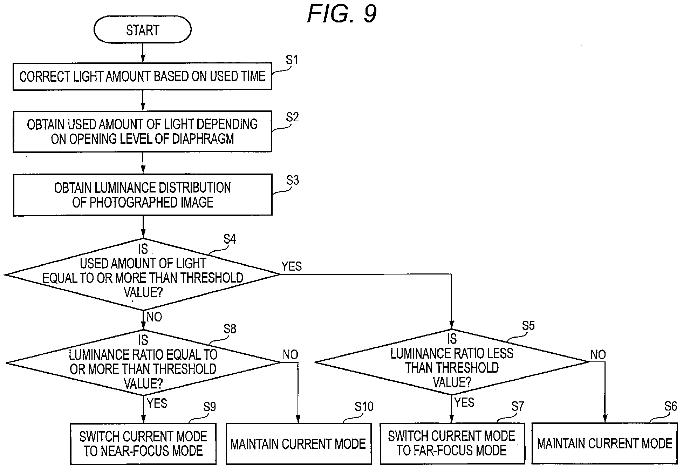

Hereinafter, the switching of the modes in actually using the endoscope device 100 will be described with reference to FIG. 9.

FIG. 9 is a flowchart illustrating a mode switching sequence of a focus distance.

The control unit 73 first sets the amount of the illumination light based on the luminance information output from the image processing unit 67 by performing imaging by the imaging element 17. In this case, the control unit 73 corrects a light amount for a lighting time by referring to a lighting time of the light source 51 measured by the timer 57 (S1).

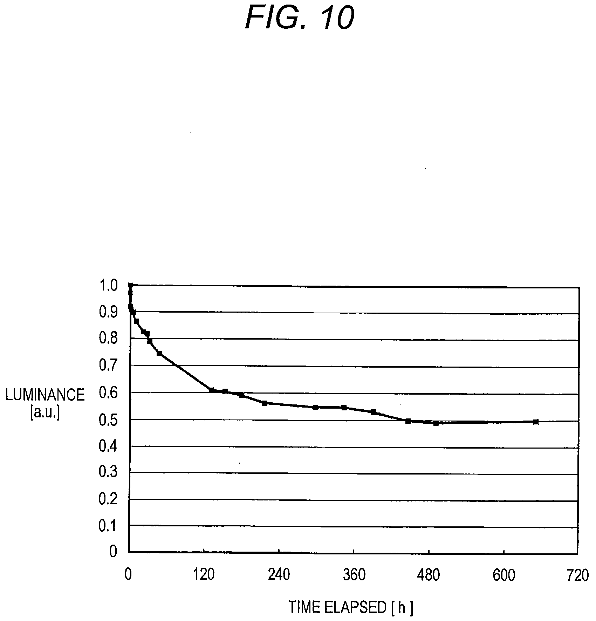

FIG. 10 is a graph illustrating a relationship between a lighting time of a light source and illuminance. The relationship between the lighting time and the illuminance illustrated in FIG. 10 is stored in the storage unit 71 in advance and the control unit 73 calculates a correction coefficient corresponding to the reduction level of the illuminance with reference to a reduction level of illuminance corresponding to a current lighting time. In addition, the control unit 73 corrects a set value by multiplying a set value for setting the amount of the illumination light by the calculated correction coefficient. As a result, the amount of the illumination light from the light source 51 is controlled to increase depending on the lighting time so as to obtain illumination light of an appropriate light amount in which an influence of deterioration of the light source is corrected. As such, the control unit 73 serves as a light amount correcting unit.

Subsequently, the control unit 73 inquires of the light amount control unit 55 about the opening level of the diaphragm 53 to obtain the opening level of the diaphragm 53 which is set at present. The amount of the illumination light used for observation is obtained from the obtained opening level of the diaphragm 53 and a light-on intensity of the light source 51 (S2).

The control unit 73 obtains the luminance information of the photographed image of the subject from the image processing unit 67 to obtain the luminance distribution of the examination line (S3).

Herein, when the amount of the used illumination light obtained in step S2 is equal to or more than the second threshold value (S4) and the luminance ratio in the luminance distribution obtained in step S3 is less than the first threshold value (S5), the control unit 73 switches the mode which is set at present into the far-focus mode (S6). Further, when the luminance ratio is equal to or more than the first threshold value, the control unit 73 maintains the mode which is set at present as it is (S7).

Meanwhile, when the used amount of the illumination light is less than the second threshold value and the luminance ratio in the luminance distribution obtained in step S3 is equal to or more than the first threshold value (S8), the control unit 73 switches the mode which is set at present into the near-focus mode (S9). Further, when the luminance ratio is less than the first threshold value, the control unit 73 maintains the mode which is set at present as it is (S10).

By repeatedly executing the algorithm, an appropriate mode corresponding to the photographing distance is automatically set. Therefore, a doctor may simply operate the endoscope since it is not needed for the doctor determine the photographing distance based on his/her experience while viewing an endoscope image nor manually switch the modes.

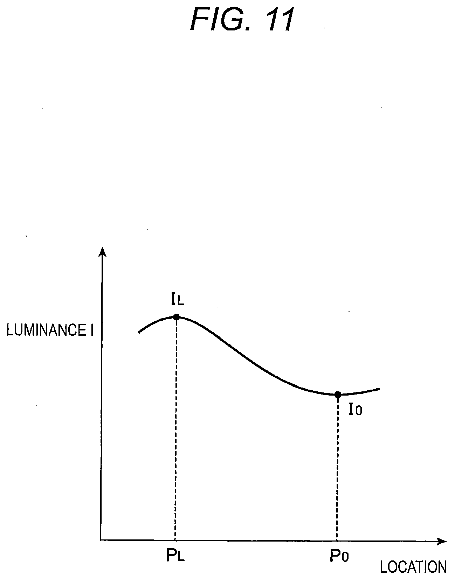

The method for estimating the photographing distance has been described above in respect to the endoscope device in which illumination windows are disposed at two locations of the front end of the endoscope insertion portion and the observation window is disposed on a straight line linking two illumination windows. However, the photographing distance may be estimated in the same manner in respect to even an endoscope device having one illumination window. FIG. 11 is a graph illustrating a luminance distribution for the examination line when the number of the illumination windows is one.

In this case, the control unit 73 obtains a location P₀ on the examination line corresponding to the location of the observation window and a luminance value of a location P_{L} on the examination line corresponding to the illumination window to obtain a luminance ratio (I_{L}/I₀) of luminance I₀ for the location P₀ and luminance I_{L} for a location P_{L}. The control unit 73 performs a mode switching using the luminance ratio. As a result, even if the number of the illumination windows is one, similarly, the mode switching depending on the photographing distance may be automatically performed.

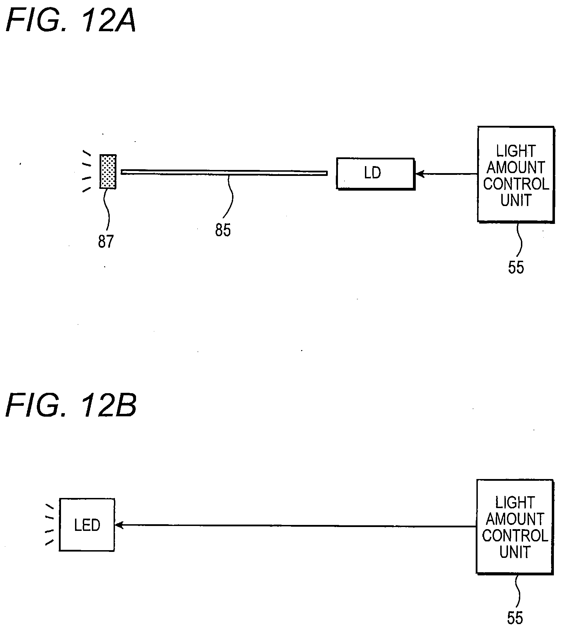

The light source device 19 is configured such that the diaphragm 53 dims light from the light source 51 but not limited thereto. For example, as illustrated in FIG. 12A, the light source device 19 may be configured by combining a laser light source LD that is driven by a pulse modulation, an optical fiber 85 (or a fiber bundle including a plurality of strands of optical fibers) that guides laser light up to an illumination window side, and an optical member 87 that includes a light diffuser or a phosphor disposed at a light emitting end of the optical fiber 85.

Further, as illustrated in FIG. 12B, an LED may be configured to be disposed at an illumination window side. Even in any case, a dimming control may be performed by a driving signal input into a light source formed by a semiconductor light emitting element to enable a light amount control which is accurate and wide in dynamic range. In addition, since the amount of emitted light varies for every individual as described above even with respect to each light source, light emitting intensity information indicating a relationship between the photographing distance up to the subject and the amount of emitted light is stored in the storage unit 71 in factory shipment of the endoscope device.

As described above, according to the endoscope device configured as described above, since the amount of the illumination light is estimated from the opening level of the diaphragm or the driving signal of the light source, it is not required to provide a new photometric sensor and thus the device configuration may be simplified.

In the case where the photographing distance is determined based on only the amount of the illumination light, it is difficult to make an accurate determination when the subject is darkened by pigment spraying. Although a correction may be performed based on the reflectivity of the pigment, the concentration of the pigment varies depending on an in-vivo state or a technician, and as a result, it is difficult to perform a feedback correction by measuring the reflectivity of the pigment. However, when the determination is made by combining the amount of the illumination light and the luminance distribution as in the present configuration, it is possible to accurately determine the photographing distance.

The present invention is not limited to the embodiments as described above. The present invention also intends to make changes and applications which may be made by those skilled in the art by combining respective components of the embodiments or based on the descriptions in the specification and well-known technologies and belong to the scope to be protected.

The present specification discloses the following:
(1) An endoscope device having illumination windows for emitting illumination light and an observation window for observing a subject, at a front end of an endoscope insertion portion inserted into an examined object, the endoscope device includes:
   a light source unit having a light source and a dimming control unit that performs dimming control of light from the light source to supply a desired light amount of illumination light to the illumination windows;
   a lens unit having a zoom tool of which a focus distance is variable to form an observation image of the subject;
   an imaging unit that photographs the observation image obtained from the lens unit to output a photographed image; and
   a focus distance switching unit that performs switching to any mode of a far-focus mode in which the focus distance of the lens unit is set at a focusing location of the subject which is farthest from the observation window and a near-focus mode in which the focus distance is set at an focusing location of the subject which is closest from the observation window according to light amount information of the illumination light and luminance distribution information of the output photographed image.
(2) The endoscope device of (1), wherein, with respect to an examination line on the photographed image corresponding to arrangement directions of the illumination windows and the observation window at the front end of the endoscope insertion portion, the focus distance switching unit detects a luminance distribution of each pixel of the examination line to obtain an inter-luminance ratio with respect to luminance values of at least two locations on the examination line,
   when the luminance ratio is equal to or more than a predetermined first threshold value and the amount of the illumination light is less than a predetermined second threshold value, the focus distance switching unit switches the current mode into the near-focus mode, and
   when the luminance ratio is less than the first threshold value and the amount of the illumination light is equal to or more than the second threshold value, the focus distance switching unit switches the current mode into the far-focus mode.
(3) The endoscope device of (1) or (2), wherein the illumination windows are disposed at a plurality of locations at the front end of the endoscope insertion portion.
(4) The endoscope device of (3), wherein the illumination windows are disposed at two locations at the front end of the endoscope insertion portion and the observation window is disposed on a straight line connecting the two illumination windows, and
   the examination line is a line on the photographed image which corresponds to a direction of the straight line.
(5) The endoscope device of (4), wherein at least one of portions to obtain the luminance ratio includes a pixel at a central position of the examination line.
(6) The endoscope device of any one of (1) to (5), wherein the light source unit includes a diaphragm that dims light from the light source, and
   the light amount information of the illumination light is information representing an opening level of the diaphragm.
(7) The endoscope device of any one of (1) to (5), wherein the light source unit dims and controls a light source of a semiconductor light emitting element by a driving signal, and
   the light amount information of the illumination light is light emitting intensity information of the semiconductor light emitting element of the driving signal.
(8) The endoscope device of any one of (1) to (7), further comprising:
   a storage unit that stores a change characteristic with time elapsed for the amount of emitted light of the light source;
   a timer that measures a lighting time of the light source; and
   a light amount correcting unit that obtains a correction coefficient corresponding to the lighting time measured by the timer with reference to the change characteristic according to the elapsed time and controls an output of the light source to increase depending on the obtained correction coefficient.

## Claims

1. An endoscope device having illumination windows (41A, 41B) for emitting illumination light and an observation window (43) for observing a subject, at a front end of an endoscope insertion portion inserted into an examined object, the endoscope device comprising:
a light source unit (51) having a light source and a dimming control unit that performs dimming control of light from the light source to supply a desired light amount of illumination light to the illumination windows;
a lens unit (45);
an imaging unit (17) that photographs the observation image obtained from the lens unit to output a photographed image;
said lens unit having a zoom tool of which a focus distance is variable to form an observation image, **characterized in that** the endoscope device further comprises
a focus distance switching unit being configured to perform (73) switching to any mode of a far-focus mode in which the focus distance of the lens unit is set at a focusing location of the subject which is farthest from the observation window and a near-focus mode in which the focus distance is set at an focusing location of the subject which is closest from the observation window according to light amount information of the illumination light and luminance distribution information of the output photographed image,
wherein, with respect to an examination line on the photographed image corresponding to arrangement directions of the illumination windows and the observation window at the front end of the endoscope insertion portion, the focus distance switching unit detects a luminance distribution of each pixel of the examination line to obtain an inter-luminance ratio with respect to luminance values of at least two locations on the examination line, to compare the luminance ratio with a predetermined threshold, so that when the luminance ratio is equal to or more than the threshold, it is determined that the subject is at the near-focus location and when the luminance ratio is less than the threshold, it is determined that the subject is at the far-focus location.

2. The endoscope device of claim 1, wherein when the luminance ratio is equal to or more than a predetermined first threshold value and the amount of the illumination light is less than a predetermined second threshold value, the focus distance switching unit switches the current mode into the near-focus mode, and
when the luminance ratio is less than the first threshold value and the amount of the illumination light is equal to or more than the second threshold value, the focus distance switching unit switches the current mode into the far-focus mode.

3. The endoscope device of claim 1 or 2, wherein the illumination windows are disposed at a plurality of locations at the front end of the endoscope insertion portion.

4. The endoscope device of claim 3, wherein the illumination windows are disposed at two locations at the front end of the endoscope insertion portion and the observation window is disposed on a straight line connecting the two illumination windows, and
the examination line is a line on the photographed image which corresponds to a direction of the straight line.

5. The endoscope device of claim 4, wherein at least one of portions to obtain the luminance ratio includes a pixel at a central position of the examination line.

6. The endoscope device of any one of claims 1 to 5, wherein the light source unit includes a diaphragm that dims light from the light source, and
the light amount information of the illumination light is information representing an opening level of the diaphragm.

7. The endoscope device of any one of claims 1 to 5, wherein the light source unit dims and controls a light source of a semiconductor light emitting element by a driving signal, and
the light amount information of the illumination light is light emitting intensity information of the semiconductor light emitting element of the driving signal.

8. The endoscope device of any one of claims 1 to 7, further comprising:
a storage unit that stores a change characteristic with time elapsed for the amount of emitted light of the light source;
a timer that measures a lighting time of the light source; and
a light amount correcting unit that obtains a correction coefficient corresponding to the lighting time measured by the timer with reference to the change characteristic according to the elapsed time and controls an output of the light source to increase depending on the obtained correction coefficient.

## Patentansprüche

1. Endoskopgerät mit Beleuchtungsfenstern (42A, 42B) zum Emittieren von Beleuchtungslicht, und einem Betrachtungsfenster (43) zum Betrachten eines Subjekts, an einem vorderen Ende eines Endoskopeinführteils, das in ein untersuchtes Objekt eingeführt ist, wobei das Endoskopgerät aufweist:
eine Lichtquelleneinheit (51) mit einer Lichtquelle und einer Dimm-Steuereinheit, die eine Dimmsteuerung von Licht aus der Lichtquelle ausführt, um eine gewünschte Menge an Beleuchtungslicht zu den Beleuchtungsfenstern zu führen;
eine Objektiveinheit (45);
eine Abbildungseinheit (17), welche das von der Objekteinheit erhaltene Betrachtungsbild fotografiert, um ein fotografiertes Bild auszugeben;
wobei die Objektiveinheit eine Zoomeinrichtung aufweist, deren Brennweite variabel ist, um ein Beobachtungsbild zu erzeugen, **dadurch gekennzeichnet, dass** das Endoskopgerät weiterhin aufweist:
eine Brennweiten-Umschalteinheit, konfiguriert zum Ausführen einer Schaltung auf einen beliebigen Modus von einem Fernfokusmodus, bei dem die Brennweite der Objektiveinheit auf eine Fokussierstelle des Patienten eingestellt ist, die am weitesten von dem Betrachtungsfenster entfernt ist, und einem Nahfokusmodus, bei dem die Brennweite auf eine Fokussierstelle des Patienten eingestellt ist, die sich in der Nähe des Betrachtungsfensters befindet, abhängig von einer Lichtmengeninformation des Beleuchtungslichts und der Luminanz-Verteilungsinformation des ausgegebenen fotografierten Bilds,
wobei in Bezug auf eine Untersuchungslinie auf dem fotografierten Bild ent - sprechend den Anordnungsrichtungen der Beleuchtungsfenster und des Betrachtungsfensters an dem vorderen Ende des Endoskopeinführteils, die BrennweitenUmschalteinheit eine Luminanzverteilung jedes Pixels der Untersuchungslinie nachweist, um ein Zwischenluminanzverhältnis zu erhalten bezüglich Luminanz - werten von mindestens zwei Stellen auf der Untersuchungslinie, um das Lumi - nanzverhältnis mit einem vorbestimmten Schwellenwert zu vergleichen, so dass, wenn das Luminanzverhältnis gleich oder größer als der Schwellenwert ist, festgestellt wird, dass das Subjekt sich an einer Nahfokusstelle befindet, während dann, wenn das Luminanzverhältnis kleiner als der Schwellenwert ist, festgestellt wird, dass das Subjekt sich an der Fernfokusstelle befindet.

2. Endoskop nach Anspruch 1, bei dem, wenn das Luminanzverhältnis gleich oder größer als ein vorbestimmter erster Schwellenwert ist und die Menge an Beleuchtungslicht geringer ist als ein vorbestimmter zweiter Schwellenwert, die Brennweiten-Umschalteinheit den laufenden Modus auf den Nahfokusmodus ein - stellt, und
wenn das Luminanzverhältnis kleiner ist als der erste Schwellenwert und die Menge an Beleuchtungslicht gleich oder größer als der zweite Schwellenwert ist, die Brennweiten-Umschalteinheit den laufenden Modus auf den Fernfokusmodus einstellt.

3. Endoskopgerät nach Anspruch 1 oder 2, bei dem die Beleuchtungsfenster an mehreren Stellen am vorderen Ende des Endoskopeinführteils angeordnet sind.

4. Endoskopgerät nach Anspruch 3, bei dem die Beleuchtungsfenster an zwei Stellen an dem vorderen Ende des Endoskopeinführteils angeordnet sind, und das Betrachtungsfenster sich auf einer die beiden Beleuchtungsfenster verbin - denden Geraden befindet, und
die Untersuchungslinie eine Linie auf dem fotografierten Bild ist, die einer Richtung der Geraden entspricht.

5. Endoskopgerät nach Anspruch 4, bei dem mindestens einer der Bereiche zum Erhalten des Luminanzverhältnisses ein Pixel an einer zentralen Stelle der Untersuchungslinie enthält.

6. Endoskopgerät nach einem der Ansprüche 1 bis 5, bei dem die Lichtquelle eine Blende enthält, welche das Licht von der Lichtquelle dimmt, und
die Lichtmengeninformation des Beleuchtungslichts Information ist, die einen Öffnungsgrad der Blende repräsentiert.

7. Endoskopgerät nach einem der Ansprüche 1 bis 5, bei dem die Lichtquelleneinheit eine Lichtquelle eines Halbleiter-Lichtemissionselements über ein Treibersignal dimmt und steuert, und
die Lichtmengeninformation des Beleuchtungslichts Lichtemissions-Intensitätsinformation des Halbleiter-Lichtemissionselements des Treibersignals ist.

8. Endoskopgerät nach einem der Ansprüche 1 bis 7, weiterhin umfassend:
eine Speichereinheit, die eine zeitliche Änderungscharakteristik für die Menge des von der Lichtquelle emittierten Lichts speichert;
einen Timer, der eine Lichtabgabezeit der Lichtquelle misst; und
eine Lichtmengen-Korrektureinrichtung, die einen Korrekturkoeffizienten ent - sprechend der von dem Timer gemessenen Lichtabgabezeit unter Bezugnahme auf die zeitliche Änderungscharakteristik ermittelt und eine Ausgangsleistung der Lichtquelle so steuert, dass diese abhängig von dem ermittelten Korrekturkoeffizienten erhöht wird.

## Revendications

1. Dispositif d'endoscope présentant des fenêtres d'éclairage (41A, 41B) destinées à émettre une lumière d'éclairage et une fenêtre d'observation (43) destinée à observer un sujet, sur une extrémité avant d'une partie d'introduction d'endoscope introduite dans un objet à examiner, le dispositif d'endoscope comprenant :
une unité de source lumineuse (51) présentant une source lumineuse et une unité de commande de gradation qui exécute une commande de gradation de la lumière provenant de la source lumineuse afin de fournir une quantité de lumière souhaitée de la lumière d'éclairage aux fenêtres d'éclairage ;
une unité formant lentille (45) ;
une unité d'imagerie (17) qui photographie l'image d'observation obtenue à partir de l'unité formant lentille afin de produire une image photographiée ;
ladite unité formant lentille présentant un outil de zoom dont la distance de mise au point est variable afin de former une image d'observation, **caractérisé en ce que** le dispositif d'endoscope comprend en outre :
une unité de commutation de distance de mise au point configurée pour exécuter (73) une commutation sur l'un quelconque de modes parmi un mode de mise au point éloigné, où la distance de mise au point de l'unité formant lentille est réglée sur un endroit de mise au point du sujet le plus éloigné de la fenêtre d'observation, et un mode de mise au point proche, où la distance de mise au point est réglée sur un endroit de mise au point du sujet qui est le plus proche de la fenêtre d'observation, en fonction des informations de quantité de lumière de la lumière d'éclairage et des informations de distribution de luminance de l'image photographiée produite,
dans lequel, par rapport à une ligne d'examen sur l'image photographiée correspondant à des directions d'agencement des fenêtres d'éclairage et de la fenêtre d'observation sur l'extrémité avant de la partie d'introduction d'endoscope, l'unité de commutation de distance focale détecte une distribution de luminance de chaque pixel de la ligne d'examen afin d'obtenir un rapport interluminance par rapport aux valeurs de luminance d'au moins deux endroits sur la ligne d'examen, afin de comparer le rapport de luminance à un seuil prédéterminé, de sorte que si le rapport de luminance est supérieur ou égal au seuil, il est déterminé que le sujet se trouve sur un endroit de mise au point proche, et si le rapport de luminance est inférieur au seuil, il est déterminé que le sujet se trouve sur un endroit de mise au point éloigné.

2. Dispositif d'endoscope selon la revendication 1, dans lequel si le rapport de luminance est supérieur ou égal à une première valeur de seuil prédéterminée et la quantité de la lumière d'éclairage est inférieure à une seconde valeur de seuil prédéterminée, l'unité de commutation de distance de mise au point commute le mode de courant sur le mode de mise au point proche, et
si le rapport de luminance est inférieur à la première valeur de seuil et la quantité de la lumière d'éclairage est supérieure ou égale à la seconde valeur de seuil, l'unité de commutation de distance de mise au point commute le mode de courant sur le mode de mise au point éloigné.

3. Dispositif d'endoscope selon la revendication 1 ou 2, dans lequel les fenêtres d'éclairage sont disposées en plusieurs endroits sur l'extrémité avant de la partie d'introduction d'endoscope.

4. Dispositif d'endoscope selon la revendication 3, dans lequel les fenêtres d'éclairage sont disposées en deux endroits sur l'extrémité avant de la partie d'introduction d'endoscope et la fenêtre d'observation est disposée sur une ligne droite reliant les deux fenêtres d'éclairage, et
la ligne d'examen est une ligne sur l'image photographiée qui correspond à une direction de la ligne droite.

5. Dispositif d'endoscope selon la revendication 4, dans lequel au moins une des parties pour obtenir le rapport de luminance inclut un pixel sur une position centrale de la ligne d'examen.

6. Dispositif d'endoscope selon l'une quelconque des revendications 1 à 5, dans lequel l'unité de source lumineuse inclut un diaphragme qui grade la lumière provenant de la source lumineuse, et
les informations de quantité de lumière de la lumière d'éclairage sont des informations représentant un niveau d'ouverture du diaphragme.

7. Dispositif d'endoscope selon l'une quelconque des revendications 1 à 5, dans lequel l'unité de source lumineuse grade et commande une source lumineuse d'un élément électroluminescent à semi-conducteurs par un signal d'excitation, et
les informations de quantité de lumière de la lumière d'éclairage sont des informations d'intensité d'émission lumineuse de l'élément électroluminescent à semi-conducteurs du signal d'excitation.

8. Dispositif d'endoscope selon l'une quelconque des revendications 1 à 7, comprenant en outre :
une unité de stockage qui stocke une caractéristique de changement avec le temps écoulé pour la quantité de lumière émise de la source lumineuse ;
une minuterie qui mesure un temps d'éclairement de la source lumineuse, et
une unité de correction de quantité de lumière qui obtient un coefficient de correction correspondant au temps d'éclairement mesuré par la minuterie en référence à la caractéristique de changement en fonction du temps écoulé et commande une sortie de la source lumineuse afin d'augmenter en fonction du coefficient de correction obtenu.
